Europäisches Patentamt

(19) European Patent Office

Office européen des brevets

(11) Veröffentlichungsnummer: **0 024 008**
A1

(12) **EUROPÄISCHE PATENTANMELDUNG**

(21) Anmeldenummer: 80104460.3

(22) Anmeldetag: 29.07.80

(51) Int. Cl.³: **A 61 C 8/00**
A 61 F 1/00

(30) Priorität: 10.08.79 DE 2932435

(43) Veröffentlichungstag der Anmeldung:
18.02.81 Patentblatt 81/7

(84) Benannte Vertragsstaaten:
AT CH DE FR GB LI

(71) Anmelder: BAYER AG
Zentralbereich Patente, Marken und Lizenzen
D-5090 Leverkusen 1, Bayerwerk(DE)

(72) Erfinder: Schulz, Hans-Hermann
Am Neulandkreuz 23
D-5653 Leichlingen(DE)

(54) Implantat für biologische Körperteile, insbesondere Zahnimplantat für den Kiefer.

(57) Implantate für biologische Körperteile, insbesondere Zahnimplantate (1), bestehen aus einem Implantatkörper (4), deren mit dem biologischen Körperteil (2) zu vereinigende Oberfläche mindestens teilweise von Fasermaterial (9) überdeckt ist, dessen Fasern vorzugsweise vornehmlich in derjenigen Richtung verlaufen, in der die Aufhängung des ursprünglichen, zu ersetzenden Körperteils bzw. dieses selbst auf Zug beansprucht worden wäre.

FIG. 1

BAYER AKTIENGESELLSCHAFT     5090 Leverkusen, Bayerwerk
Zentralbereich     Mr/Ru
Patente, Marken und Lizenzen

## Implantat für biologische Körperteile, insbesondere Zahnimplantat für den Kiefer

Die Erfindung bezieht sich auf ein Implantat für biologische Körperteile, wie Gefäße und Knochen, insbesondere Zahnimplantat für den Kiefer, bestehend aus einem Implantatkörper.

In der Implantattechnik für Knochen sind subperiostale und enossale Implantate gebräuchlich, die aus Titan, Titanlegierungen, rostfreiem Stahl, Kobalt-Chrom-Legierungen, Porzellanen oder Kompositmaterialien bestehen und vorzugsweise blattförmige, nadelförmige, zylindrische oder konische Form aufweisen und in eine anzubringende Aushöhlung des biologischen Knochenteils einsetzbar sind.

Die initiale Implantatbefestigung während der Operation erfolgt in der Regel durch direkte mechanische Fixation, z. B. mit einer Platte und Schrauben oder Nägeln. Die Befestigung kann aber auch mittels Zementen erfolgen, wie in der Hüftgelenkprothesentechnik üblich. Schließlich ist auch noch die Verkeilung mit dem Knochen bekannt.

Le A 19 166-Ausland

- 2 -

Die Erfolgsquoten sind sowohl bei subperiostalen als auch bei enossalen Implantaten stark schwankend. Eine Hauptursache für Mißerfolge sind Lockerungen der Implantate im Knochen. Diese sind auf Druckspitzen, die an kleinen Oberflächen entstehen, zurückzuführen. Diese Druckspitzen treten sowohl bei der üblichen kraftvollen Einkeilung während der Operation als auch während der Tragzeit einer Prothese auf einem Implantat auf. Die Folge davon sind Knochennekrosen, die zur Bildung von Granulationsgeweben führen.

Zur Verbindung von Blutgefäßen oder dergleichen verwendet man röhrenartige Implantate aus körperverträglichen Kunststoffen oder Metallen. Auch künstliche Herzklappen werden aus solchen Materialien hergestellt. Bei den Gefäß- und Herzklappenimplantaten besteht ebenfalls das Problem, sie mit den lebenden biologischen Körperteilen dauerhaft zu verbinden.

Die der Erfindung zugrunde liegende Aufgabe ist darin zu sehen, ein Implantat zu schaffen, das eine dauerhafte Verbindung mit dem zugehörigen biologischen Körperteil eingeht, die auch den normalen täglichen Belastungen während der gesamten Lebenszeit des Individuums standhält.

Dies Problem wird dadurch gelöst, daß wenigstens Teile der mit dem biologischen Körperteil zu vereinigenden Oberfläche des Implantatkörpers von Fasermaterial überdeckt sind. Dadurch wird erreicht, daß gegenüber den bisher üblichen Transplantaten die Verbindung zum biologischen Körperteil nicht so sehr auf schädlichen Druck, sondern durch den natürlichen Zug beansprucht wird. Dadurch wird die Gefahr von Knochennekrosen und die Bildung von Granulationsgeweben herabgesetzt.

Le A 19 166

Dies sei erklärt am Beispiel der Aufhängung eines natürlichen Zahnes im Kiefer: Der Zahn wird gehalten durch die sogenannten Sharpey'schen Fasern, die sich vom Zement der Zahnwurzel zum Alveolarknochen erstrecken. Bei der natürlichen Kaubewegung werden diese Fasern auf Zug beansprucht. Die vorliegende Erfindung macht sich dieses Naturprinzip zunutze, um durch entsprechende Gestaltung des Implantats dessen Aufhängung den natürlichen Verhältnissen anzupassen.

Das Fasermaterial besteht dabei gemäß der Erfindung aus Gewebe, Vlies, Gestrick oder Einzelfasern. Das Fasermaterial kann glatt, aufgerauht oder mikroporös sein. Soweit es sich um Gewebe, Gestrick oder Vlies handelt, gilt dies nicht nur für das Gebilde an sich, sondern vorzugsweise auch für die darin verarbeiteten Fasern. Es versteht sich, daß mehrere der genannten Eigenschaften im gesamten Fasergebilde gleichzeitig vorhanden sein können.

Als Fasermaterial eignen sich biokompatible Stoffe, wie Polyester, Polyamid, Acryl, Polyacrylnitril, Polytetrafluoräthylen, Polyäthylen, Baumwollregenerate, Kohlenstoff und Glas. Die Fasern können steif oder auch elastisch sein.

Vorzugsweise ist das Fasermaterial in einer Kleberschicht auf der Oberfläche des Implantatkörpers verankert. Hierzu eignen sich physiologisch unbedenkliche Kleber, wie sie in der Implantattechnik bereits Verwendung finden. Besonders geeignet ist ein vorzugsweise lösungsmittelfreier Zweikomponenten-Polyurethan-Kleber. Nach einer besonders vorteilhaften Ausführungsform der Erfindung ist das Fasermaterial derart auf dem Implantatkörper angeordnet, daß die Fasern des Fasermaterials

Le A 19 166

vornehmlich in derjenigen Richtung verlaufen, in der die Aufhängung des ursprünglichen, zu ersetzenden Körperteils bzw. dieses selbst bei Belastung auf Zug beansprucht worden wäre. Besteht das Fasermaterial aus Gewebe, Vlies oder Gestrick, so sind dementsprechend bevorzugt solche Gebilde zu verwenden, bei denen die Mehrzahl der Fäden bzw. Fasern die gewünschte Vorzugsrichtung einnehmen oder ihr wenigstens nahekommen.

Diese Anordnung gewährleistet eine besonders dauerhafte Verbindung des Implantats mit dem biologischen Körperteil.

Alternativ hierzu bietet sich auch an, daß Flockfasern in die Kleberschicht elektrostatisch eingebracht und ausgerichtet sind. Diese Ausführungsform ist besonders günstig, da das elektrostatische Beflockungsverfahren eine sehr genaue Ausrichtung der Einzelfasern erlaubt.

Gemäß einer weiteren Ausführungsform der Erfindung weist der Implantatkörper einen Schaft auf, der durch das Loch kegelförmiger Scheiben hindurchragt, wobei zwischen den Scheiben Fasermaterial angeordnet ist, das die Scheibenränder überragt.

Nach einer anderen Ausführungsform schließlich weist der Implantatkörper Bohrungen auf, in denen Büschel von Einzelfasern angeordnet sind.

Die beiden letzterwähnten Ausführungsformen haben den besonderen Vorteil, daß über die mit Fasermaterial zu belegende Fläche des Implantatkörpers das Fasermaterial in variabler Menge und Art verteilt werden kann. Insbesondere läßt sich auch die Faserrichtung örtlich variieren, indem beispielsweise die Scheiben der ersten

Le A 19 166

Ausführungsform unterschiedliche Neigung aufweisen, was z. B. durch variable Dicke einzelner Scheiben, über den Radius gesehen, verwirklichbar ist. Bei der zweiten Ausführungsform lassen sich die Bohrungen in unterschiedlichem Winkel zur Längsachse des Implantats und/ oder auch seitlich geneigt anbringen. Die Bohrungen können örtlich gehäuft sein, unterschiedlichen Durchmesser und/oder Neigung haben, so daß gezielt für das biologische Körperteil Anwachsflächen geschaffen werden.

Alle diese Ausführungsformen ermöglichen das Einwachsen von Knochengewebe - oder im Falle eines Gefäßimplantates das Einwachsen von Gefäßgewebe - in das Fasermaterial, ohne daß die gefürchteten Erscheinungen durch Druckbeanspruchung aufträten.

In einer Zeichnung sei das erfindungsgemäße Implantat in mehreren Ausführungsbeispielen, innerhalb eines biologischen Körperteils angeordnet, rein schematisch dargestellt und nachstehend näher erläutert. Es zeigen Fig. 1 ein Zahnimplantat im Kiefer im Schnitt, Fig. 2 ein weiteres Zahnimplantat im Kiefer, teilweise im Schnitt, Fig. 3 ein Hüftgelenkimplantat im Oberschenkel im Schnitt und Fig. 4 ein Gefäßimplantat in einem Blutgefäß im Schnitt.

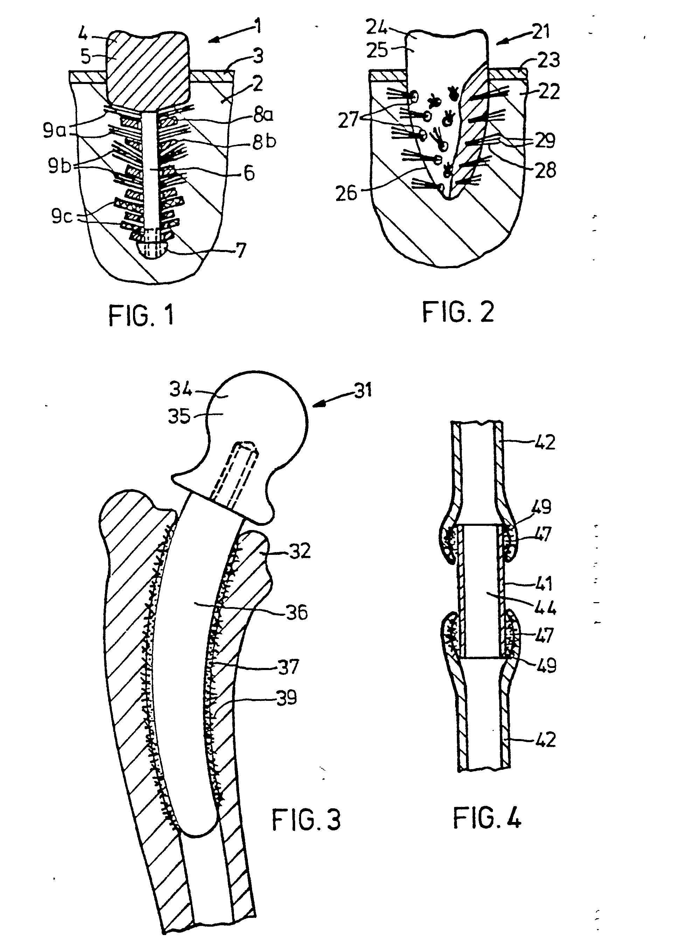

In Fig. 1 ist das Zahnimplantat 1 in vergrößertem Maßstab dargestellt und ragt in den Kieferknochen 2 hinein, der vom Zahnfleisch 3 überzogen ist. Der Implantatkörper 4 ist aus Edelstahl gefertigt und besteht aus einem nachgebildeten Zahnoberteil 5, an dem ein Schaft 6 angeordnet ist, der an seinem Ende eine Gewindekappe 7 trägt. Auf den Schaft 6 sind runde kegelstumpfförmige Scheiben 8 aus Edelstahl aufgeschoben, zwischen denen Fasermaterial 9 aus Polyamid angeordnet ist. Die

Le A 19 166

Scheiben 8a besitzen gleichmäßige Dicke, während die Scheiben 8b nach außen abnehmende Dicke aufweisen und somit einen anderen Anstellungswinkel des Fasermaterials bewirken. Das Fasermaterial 9a besteht aus Gewebe aus Polyamid, das Fasermaterial 9b aus Einzelfasern aus Kohlenstoff und das Fasermaterial 9c aus Vlies aus Polyäthylen.

In Fig. 2 ist das Zahnimplantat 21 ebenfalls in vergrößertem Maßstab dargestellt und ragt in den Kieferknochen 22 hinein, der vom Zahnfleisch 23 überzogen ist. Der Implantatkörper 24 ist aus einem Porzellan hergestellt und besteht aus dem nachgebildeten Zahnoberteil 25, das über das Zahnfleisch 23 heraussteht, und dem daran anschließenden wurzelförmigen Zapfen 26, der mit Bohrungen 27 versehen ist, in denen herausstehende Büschel 28 aus Polyamidfasern 29 angeordnet sind. Über die Oberfläche des Zapfens 26 gesehen, sind die Bohrungen 27 entsprechend den Erfordernissen unterschiedlich verteilt, haben verschiedenen Durchmesser und unterschiedliche Richtung und Neigung. Die Büschel 28 bestehen aus gemischten Fasern 29 verschiedener Stärke; sie sind teils porös, teils angerauht und teils glatt, die Köpfe der Fasern 29 sind abgerundet. Die Bohrungen 27 können auch durchgehend sein, so daß die Faserbüschel 28 quer durch den Zapfen 26 hindurchziehbar sind und ihre Enden an beiden Seiten herausragen.

In Fig. 3 ist ein Hüftgelenkimplantat 31 in verkleinertem Maßstab dargestellt und ragt in den Oberschenkelhalsknochen 32 hinein. Der Implantatkörper 34 ist aus einer Chrom-Kobalt-Legierung gefertigt und besteht aus dem nachgebildeten Hüftgelenk 35, an dem ein Schaft 36 angeordnet ist. Dessen Oberfläche ist mit einer Schicht 37 aus einem physiologisch geeigneten Kleber bedeckt. In

Le A 19 166

diese sind Flockfasern 39 aus Polyacrylnitril elektrostatisch ausgerichtet eingeflockt, deren Enden aus der
Kleberschicht 37 herausstehen und mit dem Knochen 32
verwachsen.

In Fig. 4 ist ein Blutgefäßhohlimplantat 41 in verkleinertem
Maßstab dargestellt.Die Enden des Implantatkörpers 44 sind außen
mit Kleberschichten 47 bedeckt, in denen Faservliese 49
verankert sind, die mit den darüber geschobenen Enden
des unterbrochenen biologischen Blutgefäßes 42 verwachsen sind.

Le A 19 166

- 8 -

Patentansprüche:

1. Implantat für biologische Körperteile, wie Gefäße und Knochen, insbesondere Zahnimplantat für den Kiefer, bestehend aus einem Implantatkörper, dadurch gekennzeichnet, daß wenigstens Teile der mit dem biologischen Körperteil (2, 22, 32, 42) zu vereinigenden Oberfläche des Implantatkörpers (4, 24, 34, 44) von Fasermaterial (9, 29, 39, 49) überdeckt sind.

2. Implantat nach Anspruch 1, dadurch gekennzeichnet, daß das Fasermaterial (9, 29, 39, 49) aus Gewebe, Vlies, Gestrick, Einzelfasern besteht.

3. Implantat nach Anspruch 1 und 2, dadurch gekennzeichnet, daß das Fasermaterial (9, 29, 39, 49) glatt, aufgerauht, mikroporös ist.

4. Implantat nach Anspruch 1 bis 3, dadurch gekennzeichnet, daß das Fasermaterial (9, 29, 39, 49) in einer Kleberschicht (37, 47) auf der Oberfläche des Implantatkörpers (34, 44) verankert ist.

5. Implantat nach Anspruch 3, dadurch gekennzeichnet, daß Flockfasern (39) in die Kleberschicht (37) elektrostatisch eingebracht und ausgerichtet sind.

6. Implantat nach Anspruch 1 bis 4, dadurch gekennzeichnet, daß der Implantatkörper (4, 34) einen Schaft (6) aufweist, der durch das Loch kegelstumpfförmiger Scheiben (8) hindurchragt, wobei zwischen den Scheiben (8) Fasermaterial (9) angeordnet ist, das die Scheibenränder überragt.

<u>Le A 19 166</u>

7. Implantat nach Anspruch 1, dadurch gekennzeichnet, daß der Implantatkörper (24) Bohrungen (27) aufweist, in denen Büschel (28) von Einzelfasern (29) angeordnet sind.

8. Implantat nach den Ansprüchen 1 bis 7, dadurch gekennzeichnet, daß die Fasern des Fasermaterials (9, 29, 39, 49) vornehmlich in derjenigen Richtung verlaufen, in der die Aufhängung des ursprünglichen, zu ersetzenden Körperteils bzw. dieses selbst auf Zug beansprucht worden wäre.

Le A 19 166-Ausland

FIG. 1

FIG. 2

FIG. 3

FIG. 4

| | Europäisches Patentamt | EUROPÄISCHER RECHERCHENBERICHT | Nummer der Anmeldung<br>EP 80 10 4460 |

## EINSCHLÄGIGE DOKUMENTE

| Kategorie | Kennzeichnung des Dokuments mit Angabe. soweit erforderlich. der maßgeblichen Teile | betrifft Anspruch |
|---|---|---|
| X | <u>US - A - 3 934 347</u> (LASH)<br>* Spalte 3, Zeilen 28-35;<br>Figuren 1-6 * | 1,2,4 |
| | -- | |
| X | <u>US - A - 4 084 266</u> (POIRIER)<br>* Anspruch 1; Figuren * | 1-4 |
| | -- | |
| X | <u>DE - A - 2 461 370</u> (SAUVAGE)<br>* Anspruch 1; Figuren * | 1-3 |
| | -- | |
| | <u>US - A - 3 892 648</u> (PHILLIPS)<br>* Ansprüche 3,7 * | 1,5 |
| | -- | |
| | <u>DE - A - 2 758 541</u> (BURSTEIN)<br>* Seite 13, Absatz 2; Figuren * | 1-3 |
| | -- | |
| | <u>US - A - 3 783 454</u> (SAUSSE)<br>* Anspruch 1; Figuren * | 1,2 |
| | -- | |
| P | <u>FR - A - 2 438 472</u> (SUMITO)<br>* Ansprüche * | 1-4 |
| | -- | |
| P | <u>EP - A - 0 010 865</u> (IMPERIAL)<br>* Zusammenfassung; Figuren 1,2 * | 1-3,5 |
| | ---- | |

**KLASSIFIKATION DER ANMELDUNG (Int. Cl.³)**

A 61 C 8/00
A 61 F 1/00.

**RECHERCHIERTE SACHGEBIETE (Int. Cl.³)**

A 61 C
A 61 F

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 06-11-1980 | VANRUNXT |

EPA form 1503.1 06.78